# EUROPÄISCHE PATENTANMELDUNG

(11) **EP 1 752 992 A1**
(43) Veröffentlichungstag der Anmeldung: **14.02.2007**
(21) Anmeldenummer: 05017626.2
(22) Anmeldetag: 12.08.2005
(51) Int. Cl.: G21K 1/04, A61N 5/10, H01J 37/317

(54) **Vorrichtung zur Anpassung mindestens eines Partikelstrahlparameters eines Partikelstrahls einer Partikelbeschleunigeranlage und Partikelbeschleunigeranlage mit einer derartigen Vorrichtung**

(71) Anmelder: SIEMENS AKTIENGESELLSCHAFT, 80333 München (DE)
(72) Erfinder: Grözinger, Sven, 91074 Herzogenaurauch (DE); Rietzel, Eike , Dr., 64289 Darmstadt (DE); Use, Tim, 90469 Nürnberg (DE)

(57) **Zusammenfassung**

Die Erfindung betrifft eine Partikelbeschleunigeranlage (1) zur Beschleunigung von mindestens zwei Partikelarten, wobei in einem ersten Betriebszustand eine erste Partikelart und in einem zweiten Betriebszustand eine zweite Partikelart beschleunigt werden, mit einer Vorrichtung (3) zur Anpassung eines Partikelstrahlparameters, die ein erstes von einer Halterungseinheit (25;35;41) gehaltenes Strahlformungselement aufweist, wobei das Strahlformungselement (21,23;33;42) den Partikelstrahlparameter anpasst und wobei die Halterungseinheit (25;35;41) zum Positionieren des ersten Strahlformungselements (21;23;33;42) in Abhängigkeit vom Betriebszustand ausgebildet ist, so dass das erste Strahlformungselement (21,23;33;42) von Partikeln des ersten Betriebszustands durchstrahlt und von Partikeln des zweiten Betriebszustands nicht durchstrahlt wird

## Beschreibung

Die Erfindung betrifft eine Partikelbeschleunigeranlage zur Beschleunigung von mindestens zwei Partikelarten, wobei in einem ersten Betriebszustand eine erste Partikelart und in einem zweiten Betriebszustand eine zweite Partikelart beschleunigt werden. Ferner betrifft die Erfindung eine Vorrichtung zur Anpassung eines Partikelstrahlparameters eines Partikelstrahls einer derartigen Partikelbeschleunigeranlage.

Aus EP 0 986 071 A3 ist eine Ionenstrahltherapiesystem bekannt, das zwei Ionenquellen, ein Beschleunigersystem mit einem Linearbeschleuniger und einem Synchrotron sowie ein Ionenstrahltransportsystem aufweist. Mit einem derartigen Ionenstrahltherapiesystem können verschiedene Ionensorten beschleunigt und zu Behandlungsplätzen geführt werden.

Neben dem Aufbau zweier getrennter Ionenquelle entsprechend EP 0 986 071 A3 können verschiedene Ionen auch mit einem Aufbau gewonnen werden, bei dem zwei Ionisierungsvorrichtungen, d.h. Quellen von unterschiedliche Ionensorten, ausgetauscht und abwechselnd betrieben werden. Beide Aufbauvarianten werden im Folgenden unter dem Begriff Partikelquelle verstanden.

Bei der Strahlentherapie wird bei einer Bestrahlungssitzung ein Patient an einem Behandlungsplatz positioniert und seine Positionierung in Hinblick auf die der Therapieplanung zugrunde liegende Position verifiziert. Anschließend erfolgt eine Bestrahlung aus einer oder mehreren Einfallsrichtungen mit Partikeln, beispielsweise Protonen, Pionen, Helium-, Kohlenstoff- oder Sauerstoff-Ionen.

Bei Partikeltherapieanlagen, wie sie z.B. durch die EP 0 986 071 A3 beschreiben wird, wurden bislang passive Elemente in der Regel manuell vor dem Beginn einer Bestrahlungssitzung in einem Therapiestrahlengang angebracht. So werden z.B. Ripple-Filter oder Reichweitenverschieber je nach Notwendigkeit manuell am Strahlausgang angebracht.

Ein Ripple-Filter ist beispielsweise aus der Veröffentlichung von U. Weber und G. Kraft: "Design and construction of a ripple filter for a smoothed depth dose distribution in conformal particle therapy", Phys. Med. Biol. 44 (1999) 2765-2775 bekannt.

Ferner ist beispielsweise aus der US 5,440,133 ein Streusystem für einen geladenen Partikelstrahl bekannt. Das Streusystem ist dabei derart ausgebildet, dass seine Dicke gleichmäßig und kontinuierlich einstellbar ist. Beispielsweise werden zwei Keile entgegengesetzt in den Strahl hinein oder heraus gefahren.

Verschiedene Bestrahlungsanlagen und -techniken sind von H. Blattmann in "Beam delivery systems for charged particles", Radiat. Environ. Biophys. (1992) 31:219-231 beschrieben. Darin wird u.a. auf die Verwendung von Strahlformungselementen wie Reichweitenmodulatoren, Kollimatoren und Bolus-Elementen eingegangen. Ein Bolus dient beispielsweise zur Reichenweitenanpassung der hinteren Dosisverteilung an einen Tumor. Auch in Verbindung mit einem Rasterscanverfahren können bei der Partikeltherapie passive Elemente zur Strahlformung eingesetzt werden.

Eine Aufgabe der Erfindung liegt darin, die im Arbeitsablauf beim Betreiben einer Partikelbeschleunigeranlage, insbesondere einer Partikeltherapieanlage, im Hinblick auf die Verwendung verschiedener Partikelarten zu vereinfachen.

Eine weitere Aufgabe der Erfindung liegt darin, eine partikelspezifische Anpassung eines Partikelstrahlparameters eines Partikelstrahls zu vereinfachen.

Diese Aufgabe wird erfindungsgemäß gelöst durch eine Vorrichtung zur Anpassung mindestens eines Partikelstrahlparameters eines Partikelstrahls einer Partikelbeschleunigeranlage, die mindestens in zwei Betriebszuständen betrieben werden kann, wobei in einem ersten Betriebszustand eine erste Partikelart und in einem zweiten Betriebszustand eine zweite Partikelart beschleunigt werden, wobei die Vorrichtung ein erstes von einer Halterungseinheit gehaltenes Strahlformungselement umfasst, wobei das Strahlformungselement den Partikelstrahlparameter anpasst und wobei die Halterungseinheit zum Positionieren des ersten Strahlformungselements in Abhängigkeit vom Betriebszustand ausgebildet ist, so dass das erste Strahlformungselement im ersten Betriebszustand von Partikeln durchstrahlt und im zweiten Betriebszustand nicht von Partikeln durchstrahlt wird.

Die Aufgabe bezogen auf die eingangs erwähnte Partikelbeschleunigeranlage wird durch eine Partikelbeschleunigeranlage nach Anspruch 5 gelöst.

Ein Vorteil einer erfindungsgemäßen Vorrichtung zur Anpassung eines Partikelstrahlparameters oder deren Verwendung in einer Partikelbeschleunigeranlage liegt darin, dass die manuelle und entsprechend zeitaufwändige Positionierung eines Strahlformungselements entfällt und mithilfe der Halterungseinheit und insbesondere einem Verstellmechanismus automatisiert vorgenommen werden kann. Dies erlaubt es, einen Patienten mit verschiedenen Partikelarten zu bestrahlen, ohne dass ein Austausch eines partikelspezifischen Strahlformungselements manuell vorgenommen werden muss. D.h., die Anpassung des Partikelstrahls mit Hilfe eines Strahlformungselements kann beispielsweise von einem Therapiekontrollzentrum der Partikeltherapieanlage aus veranlasst werden. Dadurch entfällt die zeitaufwändige Unterbrechung für einen Austausch von Strahlformungselementen. Dies vereinfacht den Bestrahlungsvorgang und entlastet die Überwachung des Bestrahlungsplatzes hinsichtlich der Anwesenheit von Bedienpersonal.

Bei der Verwendung einer Ausführungsform der Erfindung in Kombination mit Rasterscanverfahren wird z.B. eine Anzahl von partikelspezifischen Strahlformungselementen bereitgehalten, welche nicht patientenspezifisch sind. Die Patientenunabhängigkeit erlaubt es, die Anpassung der Partikeltherapieanlage beim Wechsel zwischen Patienten oder/und Partikelarten zu automatisieren.

In einer vorteilhaften Ausbildungsform einer Vorrichtung zur Anpassung des Strahlparameters weist diese eine automatische Antriebseinheit zur Positionierung mindestens eines Strahlformungselements auf, wobei in einer besonders vorteilhaften Ausführungsform Positionssensoren zur Positionsüberwachung des oder der Strahlformungselemente vorhanden sind.

In einer weiteren vorteilhaften Ausführungsform der Vorrichtung weist diese ein zweites, von der Halterungseinheit gehaltenes Strahlformungselement auf, das den gleichen und/oder einen anderen Strahlparameter beeinflusst, das ferner fest mit dem ersten Strahlformungselement verbunden oder unabhängig vom ersten Strahlformungselement bewegbar ist und das insbesondere derart positionierbar ist, dass das zweite Strahlformungselement im zweiten Betriebszustand von Partikeln durchstrahlt und im ersten Betriebszustand von Partikeln nicht durchstrahlt wird. Dies ermöglicht es, den Betrieb der Partikeltherapieanlage automatisiert partikelspezifisch durchzuführen, da im jeweiligen Betriebszustand nur die Strahlelemente für die jeweilige Partikelart automatisiert in den Partikelstrahl gebracht werden. D.h., diese Ausführungsform hat den Vorteil, dass bei zwei oder mehreren Strahlformungselementen die Strahlparameter mehrerer Partikelarten partikelspezifisch modifiziert und angepasst werden können. Mögliche Strahlformungselemente sind dabei beispielsweise Ripple-Filter zur Aufweitung der Partikelenergieverteilung des Partikelstrahls, ein Strahlkollimator zur Begrenzung des Partikelstrahls in seiner radialen Ausdehnung, ein Reichweitenverschieber zur Anpassung der Partikelenergie, ein Bolus zur Reichenweitenanpassung der hinteren Dosisverteilung an einen Tumor oder ein Streuelement zur Strahlaufweitung.

Weitere vorteilhafte Ausführungsformen der Erfindung sind durch die Merkmale der Unteransprüche gekennzeichnet.

Es folgt die Erläuterung von mehreren Ausführungsbeispielen der Erfindung anhand der Figuren 1 bis 6. Es zeigen:
- FIG 1: eine schematisch dargestellte Partikeltherapieanlage,
- FIG 2 und 3: eine erste Ausführungsform einer Vorrichtung zur Anpassung eines Partikelstrahlparameters,
- FIG 4 und 5: eine zweite Ausführungsform einer derartigen Vorrichtung und
- FIG 6 und 7: eine dritte Ausführungsform einer derartigen Vorrichtung.

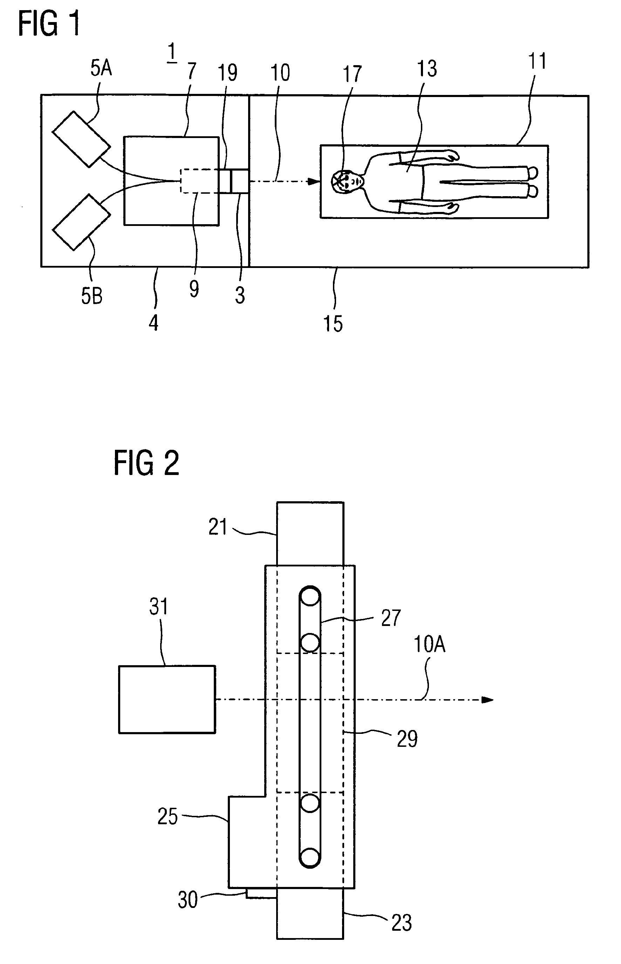

Figur 1 zeigt schematisch eine Partikeltherapieanlage 1 wie sie z.B. aus EP 0 986 071 A3 bekannt ist. In ihr wird eine Ausführungsform einer Vorrichtung 3 zur Anpassung eines Strahlparameters nach der Erfindung verwendet.

Die Partikeltherapieanlage 1 umfasst auf der Beschleunigerseite 4 zwei Partikelquellen 5A und 5B sowie eine Vakuumstrahlführungs- und Beschleunigungssystem 7. Dieses umfasst beispielhaft auch eine Rasterscanvorrichtung 9. Ein derartiger Aufbau ist darauf ausgelegt, verschiedene Partikelarten der Partikelquellen 5A und 5B auf Energien von einigen hundert MeV zu beschleunigen und mindestens einem Strahlentherapieplatz 15 zuzuführen.

Bei der Bestrahlung wird ein Partikelstrahl 10 auf einen auf einer Liege 11 positionierten Patienten 13 gerichtet. Der Strahlentherapieplatz 15 umfasst eine Patientenpositioniervorrichtung (nicht eingezeichnet), die es ermöglicht, den Patienten 13 mit dem zu bestrahlenden Gewebe in einem Isozentrum 17 des Strahlentherapieplatzes 15 zu positionieren. Der Partikelstrahl 10 durchläuft vorzugsweise in einer Nullstellung der Rasterscanvorrichtung 9 das Isozentrum 17.

Zur Strahlüberwachung umfasst die Partikeltherapieanlage 1 ferner ein Strahlüberwachungssystem 19, das beispielsweise die Strahlposition und Strahlintensität überwacht. Das Strahlüberwachungssystem 19 ist zwischen dem Patienten 13 und dem Strahlaustritt aus dem Vakuum-Strahlführungs- und Beschleunigungssystem 7 angeordnet. Die Vorrichtung 3 zur Anpassung mindestens eines Partikelstrahlparameters ist vorzugsweise dem Strahlüberwachungssystem 19 in Strahlrichtung nachgelagert. Sie erlaubt es, einen Partikelstrahlparameter auf die jeweils gerade verwendete Partikelart anzupassen, d.h., sie stellt Strahlformungselemente für verschiedene Partikelarten zur Verfügung.

Die Vorrichtung 3 wird vorzugsweise in einem kompakten Aufbau ausgeführt, um möglichst wenig Platz einzunehmen und von außen z.B. für die Wartung gut zugänglich zu sein.

Die Vorrichtung 3 erlaubt es, Strahlformungselemente partikelspezifisch in Abhängigkeit vom Betriebszustand zu positionieren, so dass z.B. ein erste Strahlformungselement von Partikeln des ersten Betriebszustands durchstrahlt und von Partikeln des zweiten Betriebszustands nicht durchstrahlt wird und dass ein zweites Strahlformungselement von Partikeln des ersten Betriebszustands nicht durchstrahlt und von Partikeln des zweiten Betriebszustands durchstrahlt wird. Dazu werden die Strahlformungselemente bei Bedarf im Partikelstrahl, z.B. im Verlauf des Partikelstrahls im Scanbereich, positioniert. Mögliche funktionelle Ausbildungen der Vorrichtung 3 werden in den folgenden Figuren anhand von Ausführungsbeispielen näher erläutert.

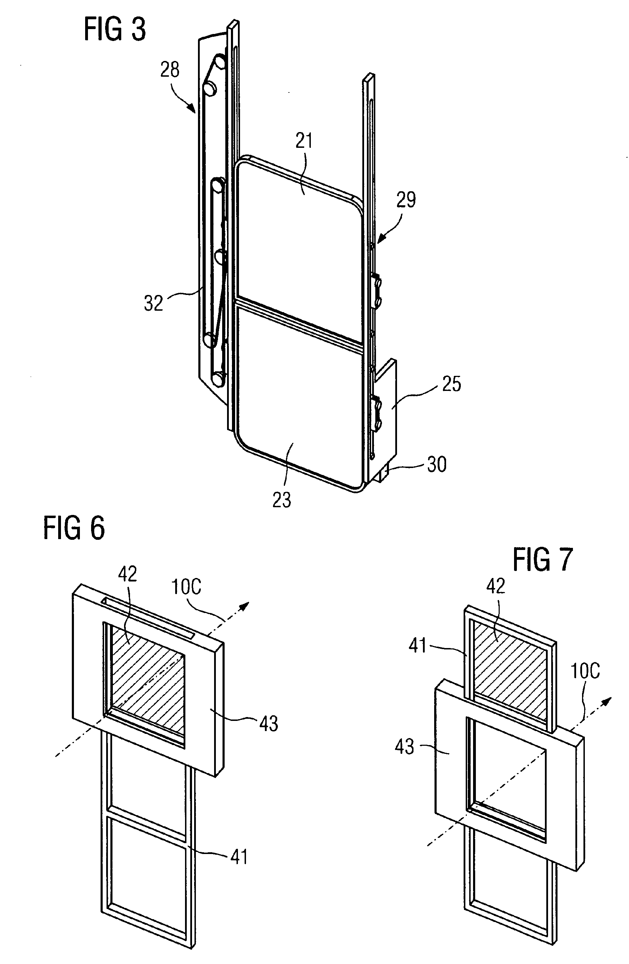

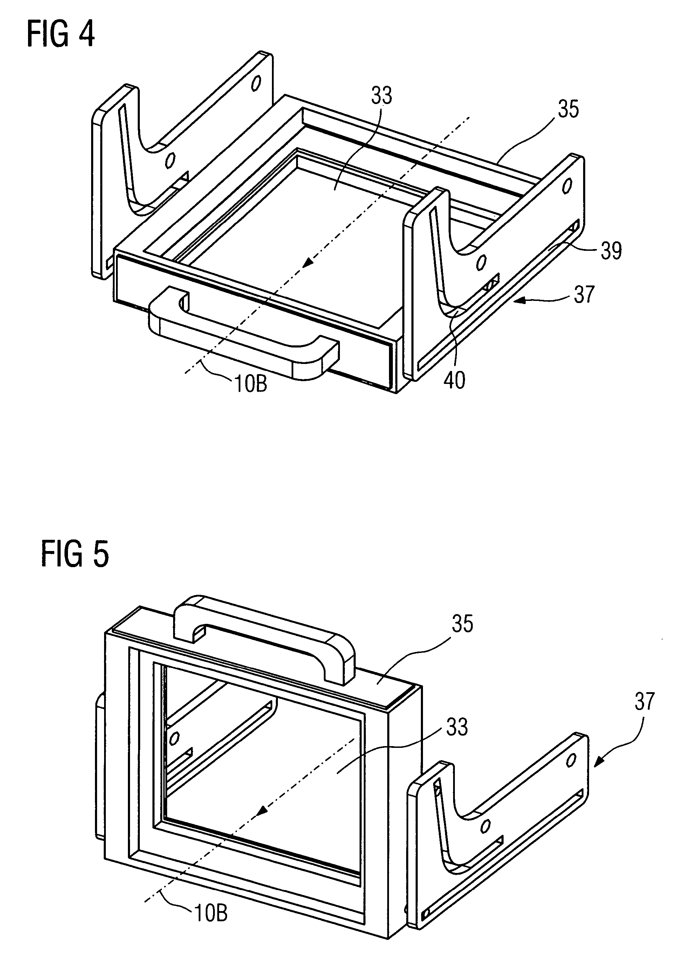

Figur 2 zeigt eine Skizze einer Vorrichtung, in der zwei Strahlformungselemente 21, 23 unabhängig voneinander in einer Ebene bewegt werden können. Die Strahlformungselemente 21, 23 werden von einer Halterungseinheit 25 gehaltert, die beispielsweise ein Führungssystem 27 aufweist. Die Strahlformungselemente 21 und 23 werden mit Hilfe einer Antriebsvorrichtung 28 (siehe Figur 3) einzeln zwischen einer Parkposition und einer Strahlposition 29 verfahren. Die Strahlformungselemente 21 und 23 befinden sich in Figur 2 jeweils in ihren Parkpositionen. Die eingenommenen Positionen werden durch Sensoren, z.B. durch einen Sensor 30, detektiert.

Ferner sind in Figur 2 schematisch ein Strahlaustritt 31 aus der Vakuumstrahlführungs- und Beschleunigungseinheit und ein Partikelstrahl 10A angedeutet. Vorzugsweise wird die tatsächliche Lage des bzw. der Strahlformungselemente 21, 23 durch mindestens einen unabhängigen Sensor kontinuierlich (z.B. mit einem Encoder) oder diskret (z.B. durch einen Endschalter, hier der Sensor 30) überwacht.

Figur 3 zeigt eine räumliche Darstellung der Vorrichtung aus Figur 2 in einem Betriebszustand, bei dem eines der Strahlformungselemente durchleuchtet wird. Dabei wurde beispielsweise das Strahlformungselement 21 mithilfe der Antriebsvorrichtung 28 in die Strahlposition 29 gebracht. Die Antriebsvorrichtung umfasst z.B. ein mit einem Elektromotor angetriebenes Riemensystem 32.

Die Figuren 4 und 5 zeigen eine weitere Ausführungsform, mit der ein Strahlformungselement 33, z.B. ein Ripple-Filter, in einen horizontal verlaufenden Partikelstrahl 10B (genauer in einen möglichen Strahlverlauf) ein- und ausgeklappt werden kann. Dazu ist das Strahlformungselement 33 in einem Rahmen 35 befestigt. In Figur 3 befindet sich das Strahlformungselement 33 in einer horizontalen Parkposition, aus der es mittels eines Kurvengetriebes 37 in eine senkrechte Strahlposition bringbar ist. Der benötigte Antrieb erfolgt beispielsweise über einen nicht abgebildeten Elektromotor durch Translation des Rahmens 35 in einer unteren Führschiene 39. Durch die Ausformung einer oberen Führschiene 40 klappt der Rahmen 35 aus der unteren horizontalen Parkposition in die in Figur 5 abgebildete aufrechte Strahlposition. Der Positionswechsel ist reversibel und kann aufgrund des kurzen Verfahrweges schnell durchgeführt werden. Diese Ausführungsform hat den Vorteil, dass sie im Wesentlichen nur in Strahlrichtung Platz benötigt. Eine Positionsüberwachung erfolgt z.B. wieder über Endschalter, die die Parkposition und die Strahlposition anzeigen. Optional kann eine kontinuierliche Positionsbestimmung über einen Encoder erfolgen.

Eine weitere Ausführungsform ist den Figuren 6 und 7 zu entnehmen. Dabei wird eine Rahmenstruktur 41 verwendet, die Platz für mindestens 1 passives Strahlformungselement 42 und evtl. auch eine Leerposition (ohne Strahlformungselement) aufweist. Vorzugsweise wird für jede Partikelart ein Strahlformungselement vorgesehen, die im Rahmen 41 einen festen Abstand zueinander aufweisen. Durch ein Verschieben der Rahmenstruktur 41 entlang einer Führung 43 werden partikelspezifisch Strahlformungselemente (oder auch kein Strahlformungselement) im Partikelstrahl positioniert. In einer vorteilhaften Ausführungsform können Rahmenbereiche, die sich außerhalb der Führung 43 befinden, seitlich weggeklappt werden. Auch hier kann ein Antrieb der Rahmenstruktur z.B. über einen Elektromotor erfolgen. Der Positionswechsel der Strahlformungselemente ist reversibel, automatisiert und schnell durchführbar. Eine Positionsbestimmung der Rahmenstruktur 41 kann ebenfalls kontinuierlich oder diskret erfolgen.

Für alle möglichen Ausführungsformen gilt, dass durch die Automatisierung des Positionswechsel- und Austauschprozesses und die Verwendung von, auf die verschiedenen Partikelarten ausgelegten, Strahlformungselemente kann eine Partikeltherapieanlage auf verschiedene Bestrahlungsmoden mit unterschiedlichen Partikelarten in kurzer Zeit eingestellt werden. Dadurch entfällt eine zeitaufwändige manuelle Intervention eines Bedieners, die Aufenthaltsdauer eines Patienten am Strahlentherapieplatz wird reduziert und der Patientendurchsatz erhöht. Ferner kann die Patientenbestrahlung partikelspezifisch hinsichtlich des Arbeitsablaufes optimiert werden, und es können Fehlmontagen von Strahlformungselementen durch die automatisierte Ansteuerung und Überwachung der Vorrichtung vermieden werden.

## Patentansprüche

1. Vorrichtung (3) zur Anpassung mindestens eines Partikelstrahlparameters eines Partikelstrahls (10;10A;10B;10C) einer Partikelbeschleunigeranlage (1), die mindestens in zwei Betriebszuständen betrieben werden kann, wobei in einem ersten Betriebszustand eine erste Partikelart und in einem zweiten Betriebszustand eine zweite Partikelart beschleunigt werden, umfassend mindestens ein erstes von einer Halterungseinheit (25;35;41) gehaltenes Strahlformungselement (21;23;33;42), wobei das Strahlformungselement (21;23;33;42) den Partikelstrahlparameter anpasst und wobei die Halterungseinheit (25;35,41) zum Positionieren des ersten Strahlformungselements (21;23;33;42) in Abhängigkeit vom Betriebszustand ausgebildet ist, so dass das erste Strahlformungselement (21,23;33;42) im ersten Betriebszustand im Partikelstrahl (10;10A;10B;10C) und im zweiten Betriebszustand nicht im Partikelstrahl (10;10A;10B;10C) angeordnet ist.

2. Vorrichtung (3) nach Anspruch 1, wobei eine automatische Antriebseinheit (28) zur Positionierung mindestens eines der Strahlformungselemente (21;23;33;42) vorhanden ist, welche insbesondere Positionssensoren (30) zur Positionsüberwachung des Strahlformungselements (21;23;33;42) aufweist.

3. Vorrichtung (3) nach Anspruch 1 oder 2, wobei ein zweites von der Halterungseinheit (25;35;41) gehaltenes Strahlformungselement (21,23;33;42) vorhanden ist, das den gleichen und/oder einen anderen Strahlparameter beeinflusst, das fest mit dem ersten Strahlformungselement (21;23;33;42) verbunden oder unabhängig vom ersten Strahlformungselement (21;23;33;42) bewegbar ist und das insbesondere derart positionierbar ist, dass das zweite Strahlformungselement (21;23,33;42) im zweiten Betriebszustand im Partikelstrahl (10;10A;10B;10C) und im ersten Betriebszustand nicht im Partikelstrahl (10;10A;10B;10C) angeordnet ist.

4. Vorrichtung (3) nach einem der Ansprüche 1 bis 3, wobei eines der Strahlformungselemente (21;23;33;42) ein Ripple-Filter zur Aufweitung der Partikelenergieverteilung des Partikelstrahls, ein Strahlkollimator, ein Reichweitenverschieber, ein Bolus oder ein Streuelement ist.

5. Partikelbeschleunigeranlage (1) zur Beschleunigung von mindestens zwei Partikelarten, wobei in einem ersten Betriebszustand eine erste Partikelart und in einem zweiten Betriebszustand eine zweite Partikelart beschleunigt werden, mit einer Vorrichtung (3) zur Anpassung eines Partikelstrahlparameters, die ein erstes von einer Halterungseinheit (25;35;41) gehaltenes Strahlformungselement (21;23;33;42) aufweist, wobei das Strahlformungselement (21;23;33;42) den Partikelstrahlparameter anpasst und wobei die Halterungseinheit (25;35;41) zum Positionieren des ersten Strahlformungselements (21;23;33;42) in Abhängigkeit vom Betriebszustand ausgebildet ist, so dass das erste Strahlformungselement (21;23;33;42) im ersten Betriebszustand in einem Partikelstrahl (10;10A;10B;10C) und im zweiten Betriebszustand nicht im Partikelstrahl (10;10A;10B;10C) angeordnet ist.

6. Partikelbeschleunigeranlage (1) nach Anspruch 5, wobei die Vorrichtung (3) zur Anpassung des Strahlparameters eine automatische Antriebseinheit (28) zur Positionierung mindestens eines der Strahlformungselemente (21;23;33;42) aufweist, welche insbesondere einen Positionssensor (30) zur Positionsüberwachung des Strahlformungselements (21;23;33;42) aufweist.

7. Partikelbeschleunigeranlage (1) nach einem der Ansprüche 5 oder 6, wobei eine erste und eine zweite Partikelquelle (5A,5B) zur Erzeugung der unterschiedlichen Partikelarten, ein Vakuum-Strahlführungs- und Beschleunigungssystem (7) zur Partikelbeschleunigung und ein Behandlungsplatz (15) zur Bestrahlung eines Patienten mit den Partikeln vorhanden sind, wobei die Vorrichtung (3) zur Anpassung des Strahlparameters patientennah, insbesondere nach dem Austreten des Partikelstrahls (10;10A;10B;10C) aus dem Vakuum-Strahlführungs- und Beschleunigungssystem (7), im Partikelstrahlverlauf angeordnet ist.

8. Partikelbeschleunigeranlage (1) nach einem der Ansprüche 5 bis 7, wobei ein zweites von der Halterungseinheit (25;35;41) gehaltenes Strahlformungselement (21;23;33;42) vorhanden ist, das den gleichen und/oder einen anderen Strahlparameter beeinflusst und das fest mit dem ersten Strahlformungselement (21;23;33;42) verbunden oder unabhängig vom ersten Strahlformungselement (21;23;33;42) bewegbar ist und das insbesondere derart positionierbar ist, dass das zweite Strahlformungselement (21;23;33;42) von Partikeln des zweiten Betriebszustand durchstrahlt und von Partikeln des ersten Betriebszustands nicht durchstrahlt wird.

9. Partikelbeschleunigeranlage (1) nach einem der Ansprüche 5 bis 8, wobei eines der Strahlformungselemente (21;23;33;42) ein Ripple-Filter zur Aufweitung der Partikelenergieverteilung des Partikelstrahls (10;10A;10B,10C), ein Strahlkollimator, ein Reichweitenverschieber, ein Bolus oder ein Streuelement ist.
